(19) Europäisches Patentamt
European Patent Office
Office européen des brevets

(11) **EP 4 360 588 A1**

(12) **EUROPEAN PATENT APPLICATION**
published in accordance with Art. 153(4) EPC

(43) Date of publication:
**01.05.2024 Bulletin 2024/18**

(21) Application number: **22828820.5**

(22) Date of filing: **24.06.2022**

(51) International Patent Classification (IPC):
*A61C 13/00* (2006.01)     *A61C 13/083* (2006.01)
*C03C 4/00* (2006.01)     *C03C 10/00* (2006.01)
*C08L 33/14* (2006.01)     *C08L 35/02* (2006.01)
*C08K 3/40* (2006.01)     *C03B 32/02* (2006.01)
*A61K 6/833* (2020.01)

(52) Cooperative Patent Classification (CPC):
**A61C 13/00; A61 13/083; A61K 6/833;
C03B 32/02; C03C 4/00; C03C 10/00; C08K 3/40;
C08L 33/14; C08L 35/02**

(86) International application number:
**PCT/KR2022/009033**

(87) International publication number:
**WO 2022/270973 (29.12.2022 Gazette 2022/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.06.2021 KR 20210083051
22.04.2022 KR 20220049877
23.06.2022 KR 20220076881**

(71) Applicant: **Hass Co., Ltd.
Gangwon-do 25452 (KR)**

(72) Inventors:
• **LIM, Hyung Bong**
**Ansan-si Gyeonggi-do 15521 (KR)**
• **KIM, Sung Min**
**Yongin-si Gyeonggi-do 16903 (KR)**
• **HA, Sung Ho**
**Ansan-si Gyeonggi-do 15521 (KR)**
• **KIM, Moon Chang**
**Gangneung-si Gangwon-do 25583 (KR)**
• **KOH, Hwan Soon**
**Anyang-si Gyeonggi-do 13910 (KR)**

(74) Representative: **Gulde & Partner
Patent- und Rechtsanwaltskanzlei mbB
Wallstraße 58/59
10179 Berlin (DE)**

(54) **BULK BLOCK FOR MANUFACTURING DENTAL PROSTHESIS**

(57) Disclosed is a bulk block for manufacturing a prosthesis having high aesthetics and processability required for one-day dental prosthetic materials, which is a dental composite bulk block comprising a glass ceramic matrix and a polymer, wherein the glass ceramic matrix consists of an amorphous glass matrix and a crystalline phase dispersed in the glass matrix, the crystalline phase comprises as a main crystalline phase at least one selected from a leucite crystalline phase and a lithium disilicate crystalline phase, and has an average particle diameter of 0.01-1.0 $\mu$m, and the polymer is included in an amount of 20-40 wt% with respect to the weight of the total bulk block. The bulk block has the advantages of improved mechanical properties, being capable of preventing microleakage, exhibiting excellent aesthetics, and enabling machining.

EP 4 360 588 A1

Fig 2

☑ Amorphous Peak. Total Area = 57470 (2448). Crystallinity = 40.10%(3.12%), FWHM-Threshold = 5.0

Two-Theta (deg)

## Description

## Technical Field

The present disclosure relates to a composite bulk block for manufacturing a dental prosthesis. More particularly, the present disclosure relates to a dental prosthetic material having improved mechanical properties, being capable of preventing microleakage, exhibiting excellent aesthetics, and enabling machining.

## Background Art

With the development of the dental industry, porcelain and metal, which are conventional prosthetic materials, are suffering from problems in physical properties or tooth aesthetics, and the market share of these materials is gradually decreasing. To replace them, the market share of materials such as glass ceramic, zirconia, and the like is increasing. Furthermore, a hot-pressing process, which is a conventional prosthesis manufacturing method, requires a long manufacturing time, so it is difficult to manufacture a prosthesis in one day, as required in the current dental market, leading to a change to CAD/CAM systems. To support this change, the effectiveness of materials for 1:1 processing that enables implantation immediately after processing without additional heat treatment of glass ceramic is also improving.

Due to the development of zirconia, glass ceramic, and the like, materials with good aesthetics and high physical properties are widely used. However, since most ceramic materials undergo crystallization heat treatment after processing, it is difficult to use them as one-day prostheses.

Moreover, a ceramic material that is currently used for 1:1 processing has a problem in that the processability thereof is low because it is used in a state where it has been already undergone crystallization, and fracture occurs in a margin portion (the boundary between the prosthesis and the tooth).

In order to solve these problems and meet the requirements of the current dental market, a composite in which organic and inorganic materials are mixed has been developed. The composite is a complementary material in which the organic material suppresses brittleness, which is the disadvantage with the inorganic material, and the inorganic material improves the physical properties of the organic material, such as low strength. Furthermore, since the composite is advantageously used for 1:1 processing that enables implantation in the oral cavity immediately after processing, new composite products are being released through continuous technical development. The composite has strength of about 150 to 200 MPa depending on the type of currently commercialized product, and has superior processability compared to conventional glass ceramics that can be used for 1:1 processing.

With regard to the manufacture of the composite, Korean Patent No. 10-1682542 discloses a dental block manufacturing method, including: preparing glass ceramic; forming a ceramic porous body by making the glass ceramic porous; charging the ceramic porous body into a vacuum chamber and firstly infiltrating a polymer into the ceramic porous body in a vacuum state; and secondarily infiltrating the polymer into the ceramic porous body before the firstly infiltrated polymer is completely cured. Here, the resulting block has a biaxial flexure strength of 100 to 150 MPa.

The glass ceramic used herein is a feldspar-based glass ceramic. Specifically, it includes 2.0 to 6.0% by weight of $N_2O$, 60.0 to 65.0% by weight of $SiO_2$, 8.0 to 15.0% by weight of $K_2O$, 0.5 to 3.0% by weight of CaO, 0.5 to 2.0% by weight of BaO, 0.2 to 1.0% by weight of $CeO_2$, 0 to 0.5% by weight of $TiO_2$, 16.0 to 19.0% by weight of $Al_2O_3$ to increase the glass transition temperature and softening point and improve the chemical durability of glass ceramic, 0 to 1.0% by weight of a coloring ingredient that affects coloring, such as brightness and saturation, and exhibits fluorescence.

In addition, Korean Patent No. 10-1609291 discloses a dental block manufacturing method, including: pulverizing glass and then melting it at a temperature of 1,400 to 1,800°C; cooling the molten glass, pulverizing it, and subjecting the pulverized glass to crystallization heat treatment at 875 to 970°C; re-pulverizing the crystallized glass subjected to the crystallization heat treatment and then subjecting it to porous heat treatment at a temperature of 700 to 840°C; and infiltrating a polymer into a porous body formed in the crystallized glass subjected to the porous heat treatment. Here, the glass melted at a temperature of 1,400 to 1,800°C includes 2.0 to 6.0% by weight of $N_2O$, 60 to 65.0% by weight of $SiO_2$, 8.0 to 15% by weight of $K_2O$, 0.5 to 3.0% by weight of CaO, 0.5 to 2.0% by weight of BaO, 0.2 to 1.0% by weight of $CeO_2$, 0 to 0.5% by weight of $TiO_2$, and 16.0 to 19.0% by weight of $Al_2O_3$.

Specifically, the glass used herein is also feldspar-based glass, and the resulting block has a biaxial flexure strength of about 100 to 150 MPa.

Meanwhile, Korean Patent No. 10-2122202 discloses a composite manufacturing method using chemical bonding between inorganic and organic materials using a silane coupling agent. Specifically, the method includes: adding a thermal initiator to a first mixture of at least two organic materials having different viscosities at 20°C to 70°C; surface-treating an inorganic material using a second mixture of ethanol and 10 to 14% by weight of an acrylic silane coupling agent; mixing the first mixture to which the thermal initiator is added and the inorganic material surface-treated with the second mixture; and curing through thermal polymerization at 100°C to 150°C.

In addition, Korean Patent No. 10-2228118 discloses a dental composite composition including glass ceramic and a curable organic material. Here, the glass ceramic has an average crystal size of 50 to 400 nm, a biaxial flexure strength of 200 to 300 MPa, and a Vickers hardness of 270 to 300 Hv.

As another example, U.S. Patent No. 7807227 discloses a composite material including a porous inorganic-non-metallic matrix and a second material and a manufacturing method thereof. The method includes: obtaining a porous inorganic-non-metallic matrix sintered body by sintering an inorganic-non-metal starting material to; obtaining a surface-modified product by coating a surface of the porous inorganic-non-metallic matrix with a coupling agent; infiltrating an organic material into the surface-modified porous inorganic-non-metallic matrix; and coagulating the organic material. This method is to obtain an isotropic composite material, in which the isotropic composite material includes the porous inorganic-non-metallic matrix with a bending strength of at least 40 MPa measured according to ISO 6 872 and the organic material that at least partially fills pores of the porous inorganic-non-metallic matrix, and the isotropic composite material has an elastic modulus of at least 25 GPa, measured according to ISO 10 477, and a bending strength of at least 100 MPa, measured according to ISO 6 872.

Japanese Patent No. 4636514 discloses a dental material that can maintain mechanical strength such as abrasion resistance and bending strength, discoloration resistance, stain resistance, and aesthetics over a long period of time, is excellent in modulus of bending elasticity and impact strength, and is suitable for processing using inexpensive CAD/CAM systems; and a manufacturing method thereof. The method is of manufacturing a dental material by impregnating porous ceramic with resin, and includes: (a) forming a mixture including ceramic powder containing network-forming oxide, intermediate oxide, and network-modifying oxide and having a mean grain size of 3.0 to 50 um, and a binder into a predetermined shape; (b) firing the formed mixture to obtain a porous ceramic block with a communicating hole; (c) infiltrating at least one coupling agent selected from a silane coupling agent, a titanate coupling agent, and a zircoaluminate coupling agent into the communication hole of the porous ceramic block under ultrasonic waves and/or reduced pressure, thereby coupling a surface of the communication hole; and (d) infiltrating a monomer and/or oligomer containing at least an ethylenic double bond into the communication hole of the coupling-treated porous ceramic block under ultrasonic waves and/or reduced pressure, followed by polymerization. As a specific example, the ceramic powder used herein is an aluminosilicate-based ceramic powder in which the network-forming oxides are $SiO_2$ and $B_2O_3$, the intermediate oxide is $Al_2O_3$, and the network-modifying oxide is $Na_2O$. By impregnating resin into the communication hole of the porous ceramic, the filling rate of inorganic materials can be increased, and mechanical strength such as abrasion resistance and bending strength, discoloration resistance, stain resistance, and aesthetics can be maintained over a long period of time. Also, because the stress within the ceramic is reduced, it is possible to provide a dental material which has excellent modulus of bending elasticity and impact strength and is also suitable for CAD/CAM systems.

In addition, there are a number of related-art documents related to methods of manufacturing a composite, which is a dental prosthesis material, but as the use of the composite as a dental prosthesis material increases, new technologies are continuously being added through research and development of superior products.

**Disclosure**

**Technical Problem**

Accordingly, the present disclosure has been made keeping in mind the above problems occurring in the related art, and an objective of the present disclosure is to provide dental bulk block that enables one-day dental prosthetic service, improves the reliability of a product against cracks, reduces microleakage, and provides improved aesthetics.

Another objective of the present disclosure is to provide a bulk block for manufacturing a prosthesis, the bulk block enabling one-day dental prosthetic service and providing ease of processing during a manufacturing process.

**Technical Solution**

One aspect of the present disclosure provides a bulk block for manufacturing a prosthesis, the bulk block including: a glass ceramic matrix; and a polymer. The glass ceramic matrix may be composed of an amorphous glass matrix and a crystalline phase dispersed in the glass matrix, the crystalline phase may include as a main crystalline phase at least one selected from a leucite crystalline phase and a lithium disilicate crystalline phase, the crystalline phase may have a mean grain size of 0.01 to 1.0 um, and the polymer may be included in an amount of 20 to 40% by weight with respect to the total weight of the bulk block.

In the bulk block according to the one aspect of the present disclosure, the crystalline phase may include as the main crystalline phase lithium disilicate, and may further include as a sub-crystalline phase lithium metasilicate.

In the bulk block according to the one aspect of the present disclosure, the crystalline phase may further include as the sub-crystalline phase at least one selected from cristobalite, tridymite, quartz, eucryptite, spodumene, virgilite, petalite, and a mixture thereof.

The bulk block according to the one aspect of the present disclosure may have a 3-point bending strength of 190 to 260 MPa, a biaxial flexure strength of 180 to 260 MPa, a Vickers hardness of 55 to 135 HV0.2, and an elastic modulus of 20 to 25 GPa.

The bulk block according to the one aspect of the present disclosure may have a diametral tensile strength of 70 to 80 MPa.

The bulk block according to the one aspect of the present disclosure may have an average light transmittance of 30 to 40% within a wavelength of 300 to 800 nm, and a maximum water absorption rate of 32 $\mu$g/mm$^3$.

In the bulk block according to the one aspect of the present disclosure, the polymer may be combined with the glass ceramic matrix through silane bonding.

In the bulk block according to the one aspect of the present disclosure, the polymer may be a cured product of a curable organic material selected from (meth)acrylate monomers and oligomers containing unsaturated double bonds.

In the bulk block according to the one aspect of the present disclosure, the curable organic material may be at least one selected from the group consisting of hydroxy ethyl methacrylate (HEMA), 2,2-bis [4-(2-hydroxy-3-methacryloyloxy propoxy) phenyl] propane (Bis-GMA), triethylene glycoldimethacrylate (TEGDMA), diurethanedimethacrylate (UDMA), urethane dimethacrylate (UDM), biphenyldimethacrylate (BPDM), n-tolyglycine-glycidylmethacrylate (NTGE), polyethylene glycol dimethacrylate (PEG-DMA), and oligocarbonate dimethacrylic esters.

In the bulk block according to the one aspect of the present disclosure, the glass matrix may include 69.0 to 75.0% by weight of $SiO_2$, 12.0 to 14.0% by weight of $Li_2O$, 2.5 to 10.5% by weight of $Al_2O_3$, 0.12 to 0.22% by weight of ZnO, 1.1 to 2.7% by weight of $K_2O$, 0.1 to 0.3% by weight of $Na_2O$, and 2.0 to 6.0% by weight of $P_2O_5$.

In the bulk block according to the one aspect of the present disclosure, the glass matrix may include 2.5 to 3.5% by weight of $Al_2O_3$.

Another aspect of the present disclosure, there is provided a method of manufacturing a bulk block for manufacturing a dental prosthesis, the method including: melting a glass composition including 69.0 to 75.0% by weight of $SiO_2$, 12.0 to 14.0% by weight of $Li_2O$, 2.5 to 10.5% by weight of $Al_2O_3$, 0.12 to 0.22% by weight of ZnO, 1.1 to 2.7% by weight of $K_2O$, 0.1 to 0.3% by weight of $Na_2O$, and 2.0 to 6.0% by weight of $P_2O_5$, water-quenching a glass melt to obtain a glass formed body of coarse size, and firstly pulverizing the glass formed body to prepare glass powder with a maximum mean grain size of less than 300 $\mu$m; subjecting the glass powder to crystallization heat treatment in a furnace from room temperature to a maximum temperature of 755 to 810°C for 30 minutes to 6 hours; pulverizing the powder subjected to the crystallization heat treatment to produce glass ceramic powder with a maximum mean grain size of less than 100 $\mu$m; and forming the glass ceramic powder into a predetermined shape.

In the method according to the other aspect of the present disclosure, the glass composition may include 2.5 to 3.5% by weight of $Al_2O_3$.

Another aspect of the present disclosure, there is provided a prosthesis, including: a glass ceramic matrix; and a polymer. The glass ceramic matrix may be composed of an amorphous glass matrix and a crystalline phase dispersed in the glass matrix, the crystalline phase may include as a main crystalline phase at least one selected from a leucite crystalline phase and a lithium disilicate crystalline phase, the crystalline phase may have a mean grain size of 0.01 to 1.0 um, and the polymer may be included in an amount of 20 to 40% by weight with respect to the total weight of the prosthesis.

In the prosthesis according to the other aspect of the present disclosure, the crystalline phase may include as the main crystalline phase lithium disilicate, and may further include as a sub-crystalline phase lithium metasilicate.

In the prosthesis according to the other aspect of the present disclosure, the crystalline phase may further include as the sub-crystalline phase at least one selected from cristobalite, tridymite, quartz, eucryptite, spodumene, virgilite, petalite, and a mixture thereof.

## Advantageous Effects

According to a composite bulk block according to the present disclosure, it is possible to provide a dental prosthetic material that has improved mechanical properties, can prevent microleakage, is aesthetically excellent, and is easy to machine.

## Description of Drawings

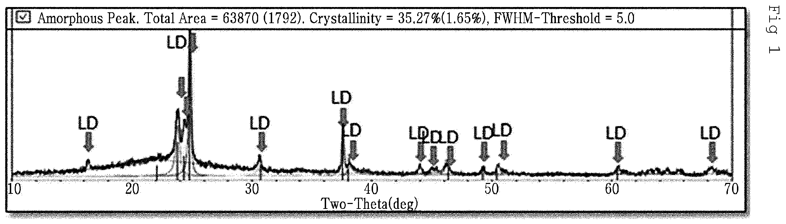

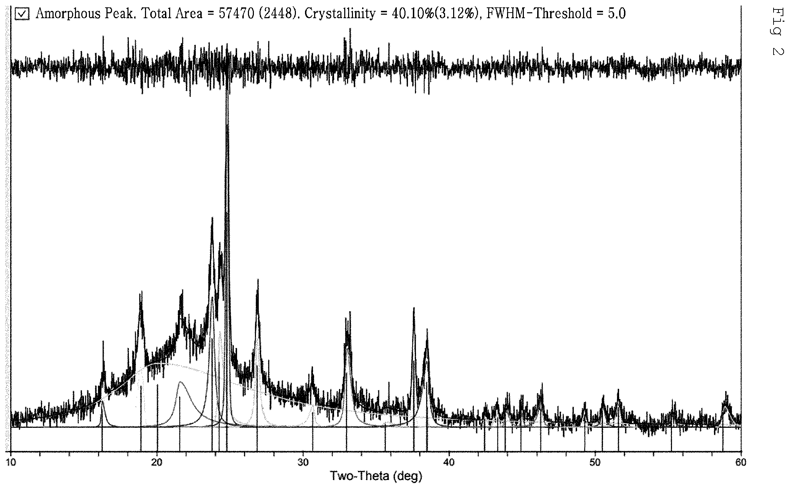

FIGS. 1 and 2 are graphs illustrating an X-ray diffraction (XRD) analysis result of bulk blocks for manufacturing prostheses according to embodiments of the present disclosure.

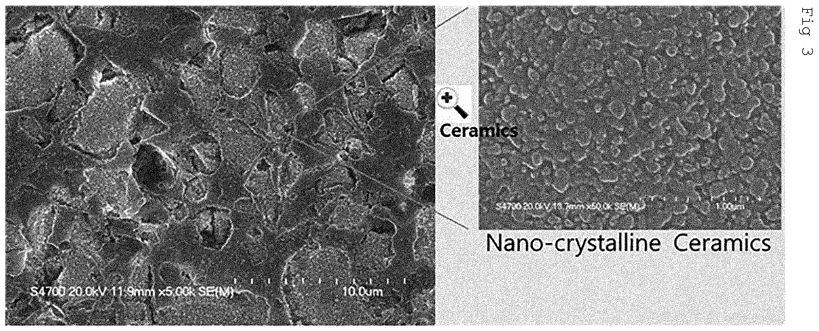

FIG. 3 is a view illustrating scanning electron microscope (SEM) images showing the microstructure and crystalline size of a bulk block for manufacturing a prosthesis according to the present disclosure.

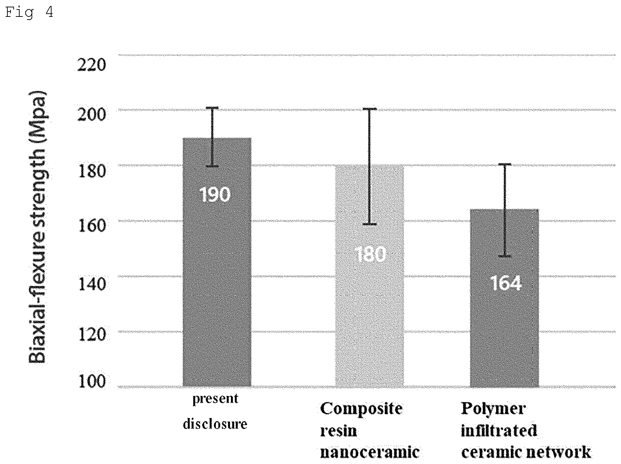

FIG. 4 is a graph illustrating the results of measurement of biaxial flexure strength of the bulk block for manufacturing the prosthesis according to the embodiment of the present disclosure by comparing with a conventional composite resin nanoceramic type product (product name: LAVA Ultimate, from 3M ESPE) and a polymer infiltrated ceramic network type product (product name: Vita Enamic, from Vita Zahnfabrik H. Rauter GmbH & Co. KG.).

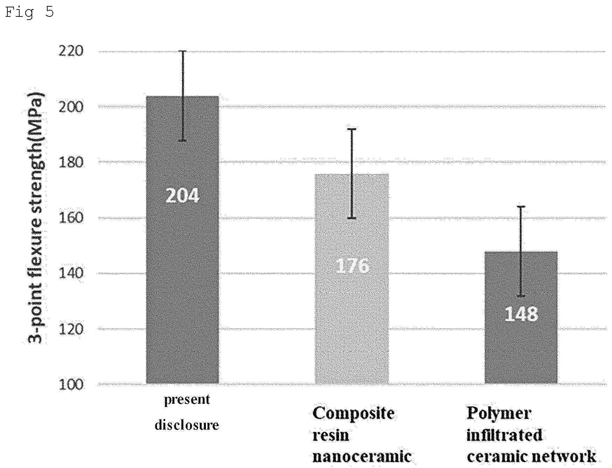

FIG. 5 is a graph illustrating the results of measurement of 3-point flexure strength of the bulk block for manufacturing the prosthesis according to the embodiment of the present disclosure by comparing with a conventional composite resin nanoceramic type product (product name: LAVA Ultimate, from 3M ESPE) and a polymer infiltrated ceramic network type product (product name: Vita Enamic, from Vita Zahnfabrik H. Rauter GmbH & Co. KG.).

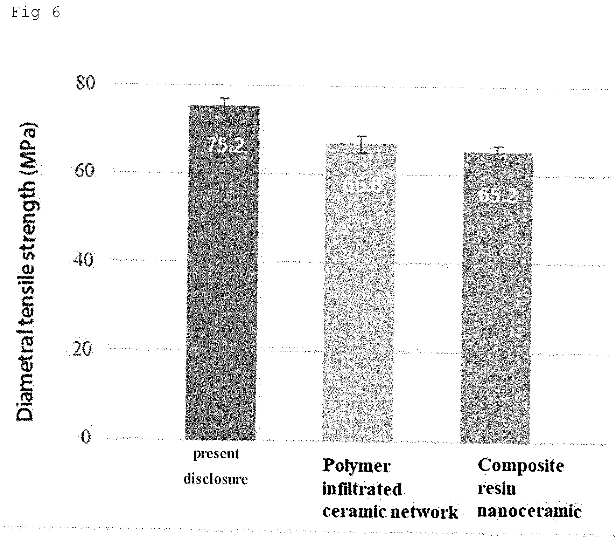

FIG. 6 is a graph illustrating the results of measurement of diametral tensile strength of the bulk block for manufacturing the prosthesis according to the embodiment of the present disclosure by comparing with a conventional composite resin nanoceramic type product (product name: LAVA Ultimate, from 3M ESPE) and a polymer infiltrated ceramic network type product (product name: Vita Enamic, from Vita Zahnfabrik H. Rauter GmbH & Co. KG.).

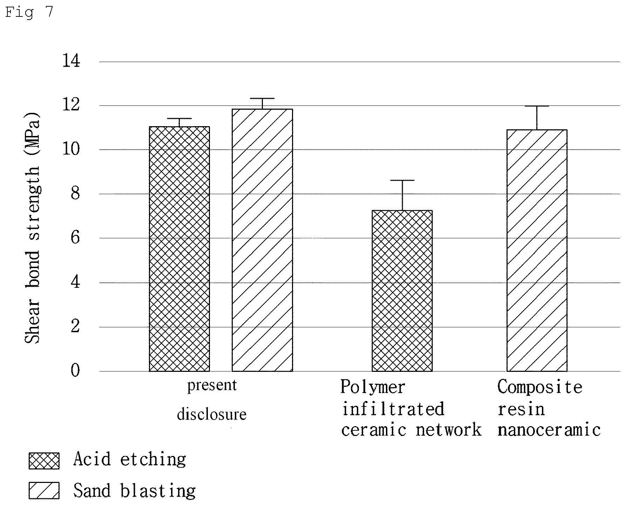

FIG. 7 is a graph illustrating the results of measurement of shear bond strength of the bulk block for manufacturing the prosthesis according to the embodiment of the present disclosure after acid etching or sandblasting by comparing with a conventional composite resin nanoceramic type product (product name: LAVA Ultimate, from 3M ESPE) and a polymer infiltrated ceramic network type product (product name: Vita Enamic, from Vita Zahnfabrik H. Rauter GmbH & Co. KG.).

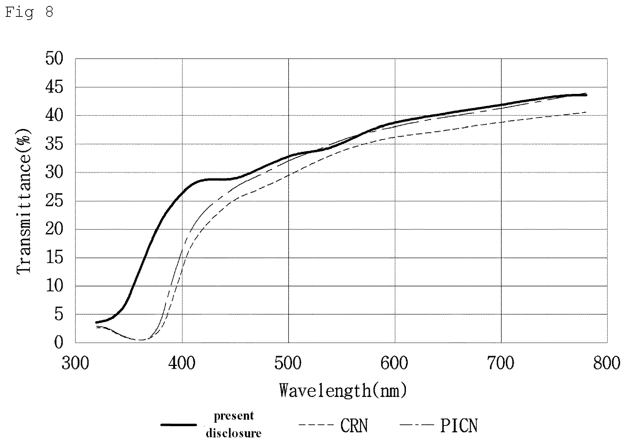

FIG. 8 is a graph illustrating the results of measurement of light transmittance of the bulk block for manufacturing the prosthesis according to the embodiment of the present disclosure by comparing with a conventional composite resin nanoceramic (CRN) type product (product name: LAVA Ultimate, from 3M ESPE) and a polymer infiltrated ceramic network (PICN) type product (product name: Vita Enamic, from Vita Zahnfabrik H. Rauter GmbH & Co. KG.).

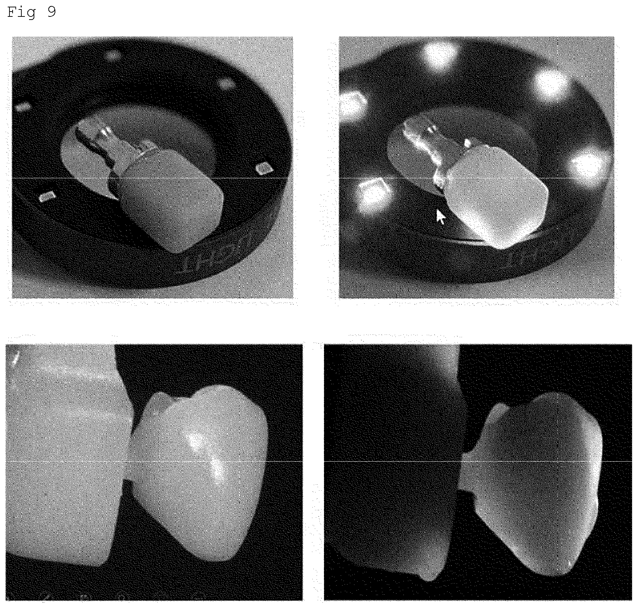

FIG. 9 is a view illustrating images showing the results that the bulk block for manufacturing the prosthesis according to the present disclosure becomes yellowish when light passes through it.

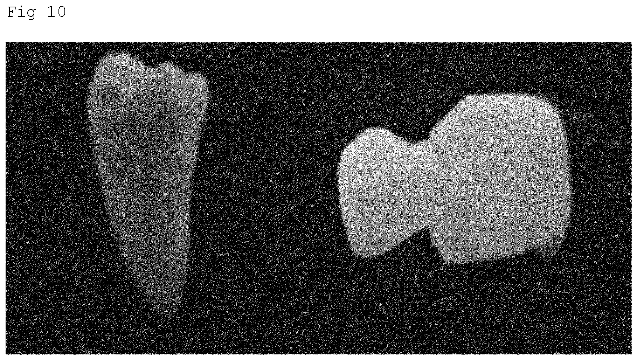

FIG. 10 is an image showing results of comparison of fluorescence between the bulk block for manufacturing the prosthesis according to the present disclosure and a natural tooth.

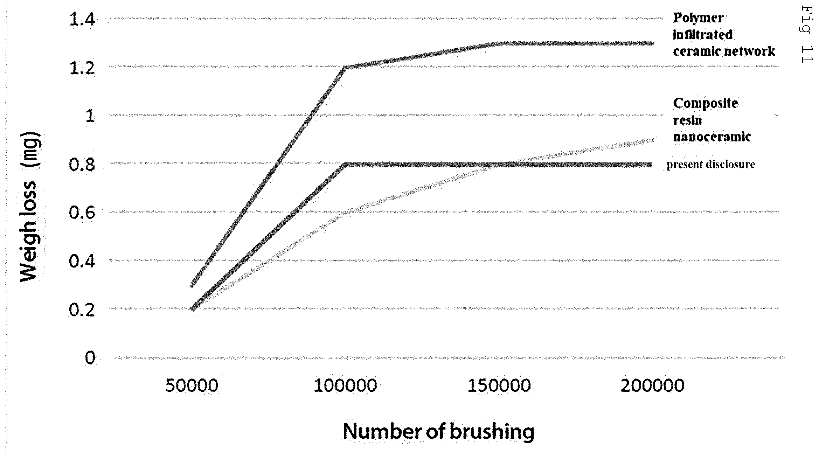

FIG. 11 is a graph illustrating the results of evaluation of wear resistance of the bulk block for manufacturing the prosthesis according to the embodiment of the present disclosure by comparing with a conventional composite resin nanoceramic (CRN) type product (product name: LAVA Ultimate, from 3M ESPE) and a polymer infiltrated ceramic network (PICN) type product (product name: Vita Enamic, from Vita Zahnfabrik H. Rauter GmbH & Co. KG.).

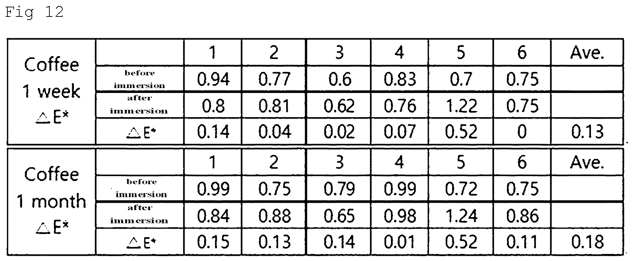

FIG. 12 is a view illustrating tables showing the results of evaluation of discoloration resistance of the bulk block for manufacturing the prosthesis according to the present disclosure.

## Best Mode

The foregoing and further aspects of the present disclosure will become more apparent from exemplary embodiments in conjunction with the accompanying drawings. Hereinafter, exemplary embodiments of the present disclosure will be described in detail such that the disclosure can be better understood and easily embodied by one of ordinary skill in the art to which this disclosure belongs.

The present disclosure provides a dental composite bulk block including a glass ceramic matrix and a polymer, in which the glass ceramic matrix is composed of an amorphous glass matrix and a crystalline phase dispersed in the glass matrix, the crystalline phase includes as a main crystalline phase at least one selected from a leucite crystalline phase and a lithium disilicate crystalline phase, the crystalline phase preferably includes as a main crystalline phase lithium disilicate in consideration of a processing process, the crystalline phase further includes as a sub-crystalline phase lithium metasilicate, the crystalline phase has a mean grain size of 0.01 to 1.0 um, and the polymer is included in an amount of 20 to 40% by weight with respect to the total weight of the bulk block.

In the previous and following descriptions, the term "main crystalline phase" may be defined as a crystalline phase occupying at least 50% by weight of the entire crystalline phase, and the term "sub-crystalline phase" may be defined as a remaining crystalline phase(s) other than the main crystalline phase in the entire crystalline phase.

The amount of the crystalline phases may be calculated through X-ray diffraction analysis. For example, in a specimen having two polymorphs a and b, the ratio Fa of the crystalline phase a is quantitatively expressed by Equation 1 below.

[Equation 1]

$$F_a = \cfrac{1}{1 + K\left(\cfrac{I_b}{I_a}\right)}$$

This value may be obtained by measuring the strength ratio of the two crystalline phases and obtaining the constant K. K is the absolute strength ratio $I_{oa}/I_{ob}$ of two pure polymorphs, which is obtained by measuring a standard material.

In the previous and following descriptions, the term "main crystalline phase" may be defined as being set on the basis of the amount calculated using this calculation method.

In the previous and following descriptions, the bulk block is not limited in shape, and for example, may include a bulk body of various types such as a block type, a disk type, an ingot type, and a cylinder type.

FIG. 1 illustrates a graph of an X-ray diffraction (XRD) analysis result of a bulk block according to an embodiment.

A bulk block for manufacturing a prosthesis according to an embodiment of the present disclosure includes a glass ceramic matrix and a polymer. According to the XRD analysis result shown in FIG. 1, a crystalline phase in glass ceramic includes lithium disilicate as the main crystalline phase, and only the pure lithium disilicate crystalline phase is precipitated in the glass matrix, with a crystallinity degree of 25 to 45%.

FIG. 2 illustrates a graph of an X-ray diffraction (XRD) analysis result of a bulk block according to an embodiment.

A bulk block for manufacturing a prosthesis according to an embodiment of the present disclosure includes a glass ceramic matrix and a polymer. According to the XRD analysis result shown in FIG. 2, a crystalline phase in glass ceramic includes lithium disilicate as the main crystalline phase and lithium metasilicate as the sub-crystalline phase. In the glass ceramic matrix according to the embodiment of the present disclosure, the crystalline phases are precipitated in the glass matrix, with a crystallinity degree of 25 to 45%.

As described above, in the case of the bulk block including the glass ceramic as a matrix, which includes lithium disilicate as the main crystalline phase and lithium metasilicate as the sub-crystalline phase, and the polymer, it can be advantageous in terms of improving processability in manufacturing bulk blocks.

In the bulk block according to the present disclosure, in addition to lithium metasilicate, the crystalline phase may include as the sub-crystalline phase at least one selected from cristobalite, tridymite, quartz, eucryptite, spodumene, virgilite, petalite, and a mixture thereof.

The glass ceramic with the above crystallinity degree may be desirable when manufacturing prostheses in consideration of transparency control through heat treatment and mechanical processability.

In the previous and following descriptions, the term "crystallinity degree" may be defined as the ratio of the crystalline phase to the amorphous glass matrix, which may be obtained through various methods. In one embodiment of the present disclosure, the crystallinity degree is a value automatically calculated by an X-ray diffractometer.

In the previous and following descriptions, XRD analysis will be understood to mean an analysis that is based on a result obtained using an X-ray diffraction analyzer (D/MAX-2500, Rigaku, Japan; CuKα (40 kV, 60 mA), scan rate: 6°/min., 2Θ: 10 to 70 (degrees)).

The crystalline phase can be formed in the form of microcrystals. These microcrystals have various sizes and size distributions depending on temperature, thereby realizing variations in mechanical properties and light transmittance.

In addition, as an example, FIG. 3 illustrates scanning electron microscope (SEM, JSM-7610F FE-SEM, JEOL) images of the dental composite bulk block according to the present disclosure, and the crystalline phase of the bulk block has a mean grain size of 0.01 to 1.0 μm.

The SEM images thus obtained may be used to calculate a mean grain size of crystalline phases. Specifically, the mean grain size may be obtained by a linear intercept method involving: drawing a diagonal line or a straight line randomly on the SEM image; dividing by the length of the line the number of crystalline grains intercepted by the line; and determining the mean grain size depending on magnification.

In the previous and following descriptions, it will be understood that the size of the crystalline phases is calculated by this method.

Meanwhile, it is preferable that the polymer present in the bulk block is included in an amount of 20 to 40% by weight with respect to the total weight of the bulk block. When the polymer is included in less than 20% by weight with respect to the total weight of the bulk block, there may be a disadvantage in use due to low processability due to the brittleness of ceramic. When the polymer is included in more than 40% by weight, problems such as fracture and wear may occur due to excessively low physical properties.

The dental composite bulk block according to the present disclosure may have a 3-point bending strength of 190 to 260 MPa, a biaxial flexure strength of 180 to 260 MPa, a Vickers hardness of 55 to 135 HV0.2, and an elastic modulus of 20 to 25 GPa.

Here, the 3-point bending strength is defined as the value measured according to ISO 6872.

The biaxial flexure strength is defined as the value measured according to ISO 4049.

The Vickers hardness is the hardness value obtained according to C1327 standard (Mitutoyo microhardness tester, Mitutoyo, Takatsu-ku, Japan), with a test load of 0.2 kgf and a holding time of 15 seconds (sec) on a specimen of 15 mm × 15 mm × 15 mm size. In the previous and following descriptions, HV0.2 is defined as the average value of the hardness values obtained by repeating the above measurement method 5 times for the same specimen.

The elastic modulus is the value measured according to ASTM E 494.

FIG. 4 is a graph illustrating the results of measurement of biaxial flexure strength of the bulk block according to the embodiment of the present disclosure by comparing with a conventional composite resin nanoceramic type product (product name: LAVA Ultimate, from 3M ESPE) and a polymer infiltrated ceramic network type product (product name: Vita Enamic, from Vita Zahnfabrik H. Rauter GmbH & Co. KG.).

FIG. 5 is a graph illustrating the results of measurement of 3-point flexure strength of the bulk block according to the embodiment of the present disclosure by comparing with a conventional composite resin nanoceramic type product (product name: LAVA Ultimate, from 3M ESPE) and a polymer infiltrated ceramic network type product (product name: Vita Enamic, from Vita Zahnfabrik H. Rauter GmbH & Co. KG.).

From the results shown in FIGS. 4 and 5, it can be found that the bulk block according to the present disclosure exhibits excellent strength compared to various types of conventional composite blocks.

The bulk block for manufacturing the prosthesis according to the present disclosure that satisfies the 3-point bending strength, biaxial flexure strength, Vickers hardness, and elastic modulus values within the above ranges do not generate cracks when manufactured into a prosthesis, thereby improving the functionality of the prosthesis, preventing fracture, and ensuring aesthetics.

Meanwhile, the bulk block for manufacturing the prosthesis according to the present disclosure may have a diametral tensile strength of 70 to 80 MPa. Here, the diametral tensile strength is measured according to ANSI/ADA 27 standard specification, which evaluates the tensile stress through a compression test that involves applying a stress load or force to the point where a material object is divided in half (across the diameter of the object). This is the result of a diametral compression test, which measures the tensile stress of a material object as the molecules of the material are pushed apart in opposite directions, similar to what happens in molecules in a direct tensile strength test.

FIG. 6 is a graph illustrating the results of measurement of diametral tensile strength of the bulk block according to the embodiment of the present disclosure by comparing with a conventional composite resin nanoceramic type product (product name: LAVA Ultimate, from 3M ESPE) and a polymer infiltrated ceramic network type product (product name: Vita Enamic, from Vita Zahnfabrik H. Rauter GmbH & Co. KG.).

As illustrated in FIG. 6, the bulk block according to the present disclosure exhibits increased tensile strength compared to various types of conventional composite blocks.

The bulk block for manufacturing the prosthesis according to the present disclosure that satisfies the diametral tensile strength value within the above range has a low possibility of microleakage. Microleakage is an osmosis phenomenon in which when a microscopic space is created between a prosthetic material and the tooth, saliva seeps into this space and then comes out again. When the occurrence of this microleakage increases, marginal discolorations may occur, marginal adaptation may decrease, and problems such as secondary caries or postoperative sensitivity may occur.

In particular, the bulk block for manufacturing the prosthesis according to the present disclosure having the diametral tensile strength value within the above range can solve the bonding problem of conventional hybrid blocks and reduce debonding.

In addition, the bulk block according to the present disclosure has excellent shear bond strength after acid etching (e.g., HF solution) or sand blasting when manufactured into a prosthesis, and this improved shear bond strength can help reduce microleakage. Specifically, the bulk block according to the present disclosure can achieve a shear bond strength of about 10 to 12 MPa after acid etching or sandblasting, so adhesion can be easily performed by selecting either acid etching or sandblasting methods depending on the user's needs.

FIG. 7 is a graph illustrating the results of measurement of shear bond strength of the bulk block according to the embodiment of the present disclosure after acid etching or sandblasting by comparing with a conventional composite resin nanoceramic type product (product name: LAVA Ultimate, from 3M ESPE) and a polymer infiltrated ceramic network type product (product name: Vita Enamic, from Vita Zahnfabrik H. Rauter GmbH & Co. KG.).

In the case of the polymer infiltrated ceramic network type product (product name: Vita Enamic, from Vita Zahnfabrik H. Rauter GmbH & Co. KG.), adhesion is improved through acid etching, and even in this case, the shear bond strength is low compared to the present disclosure.

In particular, in the case of the bulk block for manufacturing the prosthesis according to the present disclosure, the shear bond strength after sandblasting is also excellent compared to the composite resin nanoceramic type product (product name: LAVA Ultimate, from 3M ESPE).

Here, the shear bond strength is the value measured according to ISO 29022:2013.

From the results of this improved shear bond strength, it can be predicted that the bulk block according to the present disclosure can effectively reduce microleakage when manufactured into a prosthesis.

In addition, in the bulk block according to the present disclosure including a glass ceramic matrix and a polymer, in which the glass ceramic matrix is composed of an amorphous glass matrix and a crystalline phase dispersed in the glass matrix, the crystalline phase includes as a main crystalline phase at least one selected from a leucite crystalline phase and a lithium disilicate crystalline phase, the crystalline phase has a mean grain size of 0.01 to 1.0 um, and the polymer is included in an amount of 20 to 40% by weight with respect to the total weight of the bulk block, the bulk block has an average light transmittance of 30 to 40% within a wavelength of 300 to 800 nm and a maximum water absorption rate of 32 $\mu$g/mn$^3$.

Conventional polymer-ceramic-based hybrid composites have a maximum transmittance of 30% of emitted light relative to incident light. However, the bulk block according to the present disclosure can achieve an average light transmittance of up to 40% by reducing absorption and refractive index in the glass ceramic network due to the crystalline phase size of the glass ceramic. As a result, it is possible to provide a dental prosthesis with improved aesthetics compared to a dental prosthesis manufactured from a conventional hybrid composite.

In particular, since the light transmittance increases within a wavelength of 300 to 800 nm, especially at a wavelength of equal to or less than 500 nm, the time for curing using UV or LED during cementation of a prosthesis can also be shortened.

FIG. 8 is a graph illustrating the results of measurement of light transmittance of the bulk block according to the embodiment of the present disclosure by comparing with a conventional composite resin nanoceramic (CRN) type product (product name: LAVA Ultimate, from 3M ESPE) and a polymer infiltrated ceramic network (PICN) type product (product name: Vita Enamic, from Vita Zahnfabrik H. Rauter GmbH & Co. KG.).

As can be seen from FIG. 8, the bulk block for manufacturing the prosthesis according to the present disclosure exhibits high light transmittance compared to various types of conventional composite blocks in the 300 to 800 nm wavelength range, and especially it exhibits significantly high light transmittance in the 300 to 400 nm wavelength range.

In addition, the bulk block according to the present disclosure has opalescence, so it appears yellowish when light passes through it, thereby improving aesthetics. Also, in terms of fluorescence, it of course achieves a level of fluorescence comparable to that of other hybrid products.

FIG. 9 is a view illustrating images showing the results that the bulk block for manufacturing the prosthesis according to the present disclosure becomes yellowish when light passes through it. FIG. 10 is an image showing results of comparison of fluorescence between the bulk block for manufacturing the dental prosthesis according to the present disclosure and a natural tooth.

From the results shown in FIG. 9, it can be confirmed that the bulk block according to the present disclosure has an opalescence with respect to transmitted light, and from the results shown in FIG. 10, the bulk block according to the present disclosure exhibits similar fluorescence to a natural tooth, and thus it can be predicted that aesthetics can be provided to a prosthesis.

All natural teeth covered with enamel have an inherent opalescence, so they appear blue in reflected light and yellow in transmitted light. The bulk block for manufacturing the prosthesis according to the present disclosure can reproduce the opalescence and fluorescence of natural teeth.

Meanwhile, the bulk block according to the present disclosure satisfies a water absorption rate of up to 32 $\mu g/mm^3$, preferably 20 to 32 $\mu g/mm^3$. With this range, the production of hydrolyzed methacrylic acid from the polymer in the bulk block can be reduced, thereby improving the color stability of a dental prosthesis and improving the ability to repair.

Here, the water absorption rate is the value measured according to ISO 10477, Article 7.8 (Water Absorption & Solubility).

In addition, the bulk block according to the present disclosure exhibits improved tooth brushing wear resistance. This is believed due to the fact that the weight loss due to tooth brushing wear of the bulk block is reduced because the size of the crystalline phase constituting the glass ceramic network of the bulk block is small. Specifically, even after brushing more than 200,000 times, the weight loss is less than 0.8 $\mu g$.

FIG. 11 is a graph illustrating the results of evaluation of wear resistance of the bulk block according to the embodiment of the present disclosure by comparing with a conventional composite resin nanoceramic (CRN) type product (product name: LAVA Ultimate, from 3M ESPE) and a polymer infiltrated ceramic network (PICN) type product (product name: Vita Enamic, from Vita Zahnfabrik H. Rauter GmbH & Co. KG.).

The wear resistance is the value measured by counting the number of brushings for each specimen according to ISO/TR 14569-1:2007(E) standard. Each composite block was brushed up to 200,000 times, and its weight was measured every 50,000 times. At 100,000 brushings, the polymer infiltrated ceramic network (PICN) type product (product name: Vita Enamic, from Vita Zahnfabrik H. Rauter GmbH & Co. KG.) has less wear compared to the bulk block according to the present disclosure or another type of conventional composite bulk block. At 200,000 brushings, the bulk block according to the present disclosure exhibits the least wear.

From these results, it can be found that the bulk block according to the present disclosure can provide a prosthesis with less wear during masticatory movements. In the case of conventional composite blocks, strength and mechanical properties have been improved to some extent compared to ceramic or zirconia, but there have been limitations in terms of wear resistance. However, the bulk block according to the present disclosure exhibits wear characteristics that can overcome the limitations of conventional composite prosthesis.

Meanwhile, since the bulk block for manufacturing the prosthesis according to the present disclosure satisfies various physical properties as described above, discoloration resistance can also be improved. According to ISO 6872 standard, a specimen was prepared in the form of a disk with a thickness of 1.2 mm ($\pm$0.2 mm) and a diameter of 16 mm, and was immersed in a coffee solution (200 ml of regular Americano) for 1 week and 1 month. Here, a container containing the solution was stored in a constant temperature water bath, and then the specimen was taken out and washed with distilled water for 5 minutes. After that, the color difference value ($\Delta E$) before and after immersion in the coffee solution was calculated using the CIE colorimetric system of the International Commission on Illumination.

FIG. 12 illustrates tables showing the results of evaluation of discoloration resistance.

The bulk block for manufacturing the prosthesis according to the present disclosure has an average $\Delta E$ value of 0.13 and 0.18 after immersion for 1 week and 1 month, respectively. Generally, when the $\Delta E$ value is equal to or less than 0.5, no color difference is noticeable and the discoloration resistance can be said to be excellent. Thus, it can be found that the bulk block according to the present disclosure has high discoloration resistance.

The bulk block for manufacturing the prosthesis according to the present disclosure, which satisfies such various characteristics, can be used for applications such as inlays, onlays, veneers, or crowns, and is especially suitable for cutting processing such as CAD/CAM processing, making it possible to manufacture a prosthesis in one day.

In addition, the bulk block according to the present disclosure is a composite block based on glass ceramic with a microcrystalline phase, and various shades can be realized by changing crystallization heat treatment conditions during a manufacturing process.

In addition, the bulk block according to the present disclosure is a composite block based on glass ceramic with a microcrystalline phase, and various shades can be realized by changing crystallization heat treatment conditions during a manufacturing process.

Meanwhile, in the bulk block for manufacturing the prosthesis according to the present disclosure, the polymer may be combined with the glass ceramic matrix through silane bonding.

Such silane bonding may be achieved through surface treatment of the glass ceramic matrix. Specifically, a surface of the glass ceramic may be treated with organofunctional silane having an ethylenically unsaturated double bond and bonding a polymer to it.

More specifically, the organofunctional silane may be at least one selected from the group consisting of methacryloxyalkylene trialkoxysi lane, 3-methacryloxypropyl trimethoxysilane, and 3-methacryloxypropyl triethoxysilane, but is not limited thereto.

Example of such a treatment method include those disclosed in Korean Patent No. 10-1609291, Korean Patent 10-1682542, Korean Patent 10-2122202, and Korean Patent No. 10-2228118.

The polymer included in the bulk block according to the present disclosure may be a cured product of a curable organic material selected from (meth)acrylate-based monomers and oligomers containing unsaturated double bonds. A specific example may be at least one selected from the group consisting of hydroxy ethyl methacrylate (HEMA), 2,2-bis [4-(2-hydroxy-3-methacryloyloxy propoxy) phenyl] propane (Bis-GMA), triethylene glycoldimethacrylate (TEGDMA), diurethanedimethacrylate (UDMA), urethane dimethacrylate (UDM), biphenyldimethacrylate (BPDM), n-tolyglycine-glycidylmethacrylate (NTGE), polyethylene glycol dimethacrylate (PEG-DMA), and oligocarbonate dimethacrylic esters.

Among these monomers and/or oligomers, for example, UDMA or Bis-GMA have high viscosity, so they may be mixed with TEGDMA having low viscosity at a mass ratio of 5:5 to 6:4, but the present disclosure is not limited thereto.

In the case of the curable organic material, shrinkage hardening occurs during the polymerization reaction, and thus the use of the organofunctional silane is effective at minimizing a change in physical properties caused by shrinkage hardening.

The surface treatment of the glass ceramic using organofunctional silane may be performed using a solution of organofunctional silane diluted in ethanol in consideration of the specificity of a dental composite.

When the surface of the glass ceramic is treated with organofunctional silane to form a composite bulk block, the vol% of the inorganic material with respect to the total volume of the composite may increase, making it possible to increase biaxial flexure strength and hardness.

Meanwhile, the bulk block may include an initiator in order to crosslink the curable organic material into a polymer and cure the same. Examples of the initiator include a photoinitiator and a thermal initiator. In the present disclosure, a thermal initiator is preferably used, and it is possible to obtain a composite having superior physical properties when performing thermal polymerization using the thermal initiator compared to when performing photopolymerization using the photoinitiator.

The thermal initiator may include various compounds known in the art, and examples thereof include, but are not limited to, known peroxides such as dibenzoyl peroxide, dilauroyl peroxide, tert-butyl peroctoate, and tert-butyl perbenzoate.

In obtaining the bulk block for manufacturing the prosthesis according to the present disclosure as described above, the glass matrix preferably includes 69.0 to 75.0% by weight of $SiO_2$, 12.0 to 14.0% by weight of $Li_2O$, 2.5 to 10.5% by weight of $Al_2O_3$, 0.12 to 0.22% by weight of $ZnO$, 1.1 to 2.7% by weight of $K_2O$, 0.1 to 0.3% by weight of $Na_2O$, and 2.0 to 6.0% by weight of $P_2O_5$. Here, the glass matrix preferably includes 2.5 to 3.5% by weight $Al_2O_3$, considering the main crystalline phase.

This glass composition is subjected to crystal nucleation and crystal growth heat treatment for crystallization to precipitate a crystalline phase in an amorphous glass matrix. The temperature at which crystal nucleation and crystal growth occur in the glass matrix is in the range of 500 to 880°C. That is, a crystal nucleus starts to form from a minimum of 500°C and a crystal grows while the temperature is raised. The crystal grows up to a maximum of 880°C at which the bulk block exhibits the lowest light transmittance for use as the material for artificial teeth. In other words, the translucency is gradually lowered from the temperature at which the crystal starts to grow to a maximum of 880°C. Considering this phenomenon, the crystal may be grown to a degree that satisfies high strength and machinability to be used as a glass ceramic for the dental composite bulk block according to the present disclosure.

From this point of view, the glass ceramic constituting the bulk block according to the present disclosure is preferably obtained by: melting a glass composition including 69.0 to 75.0% by weight of $SiO_2$, 12.0 to 14.0% by weight of $Li_2O$, 2.5 to 10.5% by weight of $Al_2O_3$, 0.12 to 0.22% by weight of $ZnO$, 1.1 to 2.7% by weight of $K_2O$, 0.1 to 0.3% by weight of $Na_2O$, and 2.0 to 6.0% by weight of $P_2O_5$, water-quenching a glass melt to obtain a glass formed body of coarse size, and firstly pulverizing the glass formed body to prepare glass powder with a maximum mean grain size of less than 300 $\mu$m; subjecting the glass powder to crystallization heat treatment in a furnace from room temperature to a maximum temperature of 755 to 810°C for 30 minutes to 6 hours; pulverizing the powder subjected to the crystallization heat treatment to produce glass ceramic powder with a maximum mean grain size of less than 100 $\mu$m; and forming the glass ceramic powder into a predetermined shape. Here, the glass composition preferably includes 2.5 to 3.5% by weight of $Al_2O_3$, considering the main crystalline phase.

## Mode for Invention

In a specific embodiment for obtaining a glass ceramic constituting a composite bulk block according to the present disclosure, first, a glass composition is prepared by weighing and mixing 69.0 to 75.0% by weight of $SiO_2$, 12.0 to 14.0% by weight of $Li_2O$, 2.5 to 3.5% by weight of $Al_2O_3$, 0.12 to 0.22% by weight of ZnO, 1.1 to 2.7% by weight of $K_2O$, 0.1 to 0.3% by weight of $Na_2O$, and 2.0 to 6.0% by weight of $P_2O_5$.

As a component of the glass composition, $Li_2CO_3$ may be added instead of $Li_2O$. In this case, carbon dioxide ($CO_2$), which is a carbon (C) component of $Li_2CO_3$, is exhausted in a gas form during glass melting. In addition, as alkali oxides, $K_2CO_3$ and $Na_2CO_3$ may be added instead of $K_2O$ and $Na_2O$, respectively. In this case, carbon dioxide ($CO_2$), which is a carbon (C) component of $K_2CO_3$ and $Na_2CO_3$, is exhausted in a gas form during glass melting.

The mixing is performed using a dry mixing process. Specifically, a ball-milling process may be used as the dry mixing process. Specifically, the ball-milling process involves charging a starting raw material into a ball-milling machine, and then rotating the ball-milling machine at a predetermined speed to mechanically pulverize and uniformly mix the starting raw material. Balls for use in the ball-milling machine may be made of a ceramic material such as zirconia or alumina. The balls may have the same sizes, or at least two different sizes. The sizes of the balls, milling time, rotation speed of the ball-milling machine, etc. are controlled in consideration of a desired grain size. For example, in consideration of the desired grain size, the size of the balls may be set to be in the range of about 1 to 30 mm, and the rotation speed of the ball-milling machine may be set to be in the range of about 50 to 500 RPM. The ball milling is preferably performed for 1 to 48 hours in consideration of the desired grain size. During the ball milling, the starting raw material is pulverized into fine grains with a uniform grain size, and at the same time is uniformly mixed.

The mixed starting raw material is placed in a melting furnace, and then melted by heating the melting furnace containing the starting raw material therein. Here, the term "melting" means that the starting raw material is converted into a viscous liquid state, not a solid state. The melting furnace is preferably made of a material having a high melting point and a high strength and also having a low contact angle for suppressing the phenomenon in which a molten material is adhered thereto. To this end, preferably, the melting furnace is made of a material such as platinum (Pt), diamond-like carbon (DLC), or chamotte, or is coated with a material such as platinum (Pt) or diamond-like carbon (DLC).

The melting is preferably performed at 1,400 to 2,000°C under normal pressure for 1 to 12 hours. When the melting temperature is less than 1,400°C, the starting raw material may fail to melt completely. On the other hand, when the melting temperature exceeds 2,000°C, excessive energy consumption is necessary, which is not economical. Therefore, it is preferable that the melting is performed at a temperature within the above range. In addition, when the melting time is very short, the starting raw material may fail to sufficiently melt. On the other hand, when the melting time is very long, excessive energy consumption is necessary, which is not economical. The temperature increase rate of the melting furnace is preferably about 5 to 50°C/min. When the temperature increase rate of the melting furnace is very slow, a long period of time may be taken, which may reduce productivity. On the other hand, when the temperature increase rate thereof is very fast, a rapid temperature increase may cause an increase in volatilization amount of the starting raw material, and the physical properties of glass ceramic may be poor. Therefore, it is preferable that the temperature of the melting furnace is increased at a rate within the above range. The melting is preferably performed in an oxidation atmosphere such as oxygen ($O_2$) and air.

In order to pulverize a glass melt into a desired shape and size, the glass melt is water-quenched to obtain a glass formed body of coarse size, and then the glass formed body thus obtained is firstly pulverized to prepare glass powder with a maximum mean grain size of less than 300 $\mu$m.

The glass powder thus obtained is transferred to a firing furnace for crystallization heat treatment to produce desired crystallization heat-treated powder.

Here, the crystallization heat treatment is performed in the furnace for 30 minutes to 6 hours from room temperature to a maximum temperature of 755 to 810°C. As a result, a crystallization heat-treated powder including only pure lithium disilicate as a crystalline phase having a size of 0.01 to 1.0 um is obtained.

Thereafter, the crystallization heat-treated powder is pulverized to produce glass ceramic powder with a maximum mean grain size of less than 100 $\mu$m.

Finally, the glass ceramic powder is formed into a predetermined shape to obtain a glass ceramic matrix of the bulk block according to the embodiment of the present disclosure.

By using the formed product thus obtained as the glass ceramic matrix, through a method disclosed in Korean Patent No. 10-1609291, Korean Patent 10-1682542, Korean Patent 10-2122202, or Korean Patent No. 10-2228118, it is possible to manufacture a bulk block including a glass ceramic matrix and a polymer, in which the glass ceramic matrix is composed of an amorphous glass matrix and a crystalline phase dispersed in the glass matrix, the crystalline phase includes as a main crystalline phase at least one selected from a leucite crystalline phase and a lithium disilicate crystalline phase, the crystalline phase has a mean grain size of 0.01 to 1.0 um, and the polymer is included in an amount of 20 to 40% by weight with respect to the total weight of the bulk block.

The obtained bulk block may be machined into a desired shape by processing using CAD-CAM and manufactured into a prosthesis.

The prosthesis thus obtained includes a glass ceramic matrix and a polymer, in which the glass ceramic matrix is composed of an amorphous glass matrix and a crystalline phase dispersed in the glass matrix, the crystalline phase includes as a main crystalline phase at least one selected from a leucite crystalline phase and a lithium disilicate crystalline phase, the crystalline phase has a mean grain size of 0.01 to 1.0 um, and the polymer is included in an amount of 20 to 40% by weight with respect to the total weight of the prosthesis.

The prosthesis thus obtained may have a 3-point bending strength of 190 to 260 MPa, a biaxial flexure strength of 180 to 260 MPa, a Vickers hardness of 55 to 135 HV0.2, and an elastic modulus of 20 to 25 GPa. In addition, the prosthesis thus obtained may have a diametral tensile strength of 70 to 80 MPa.

In addition, the prosthesis thus obtained may have an average light transmittance of 30 to 40% within a wavelength of 300 to 800 nm, and a maximum water absorption rate of 32 $\mu$g/mm$^3$.

Although the exemplary embodiments of the present disclosure have been disclosed for illustrative purposes, those skilled in the art will appreciate that various modifications, additions, and substitutions are possible, without departing from the scope and spirit of the present disclosure as disclosed in the accompanying claims.

**Industrial Applicability**

The present disclosure relates to a bulk block for manufacturing a dental prosthesis. More particularly, the present disclosure relates to a dental prosthetic material having improved mechanical properties, being capable of preventing microleakage, exhibiting excellent aesthetics, and enabling machining.

**Claims**

1. A bulk block for manufacturing a prosthesis, the bulk block comprising:

   a glass ceramic matrix; and
   a polymer,
   wherein the glass ceramic matrix is composed of an amorphous glass matrix and a crystalline phase dispersed in the glass matrix,
   the crystalline phase comprises as a main crystalline phase at least one selected from a leucite crystalline phase and a lithium disilicate crystalline phase,
   the crystalline phase has a mean grain size of 0.01 to 1.0 $\mu$m, and
   the polymer is included in an amount of 20 to 40% by weight with respect to the total weight of the bulk block.

2. The bulk block of claim 1, wherein the crystalline phase comprises as the main crystalline phase lithium disilicate, and further comprises as a sub-crystalline phase lithium metasilicate.

3. The bulk block of claim 2, wherein the crystalline phase further comprises as the sub-crystalline phase at least one selected from cristobalite, tridymite, quartz, eucryptite, spodumene, virgilite, petalite, and a mixture thereof.

4. The bulk block of claim 1, wherein the bulk block has a 3-point bending strength of 190 to 260 MPa, a biaxial flexure strength of 180 to 260 MPa, a Vickers hardness of 55 to 135 HV0.2, and an elastic modulus of 20 to 25 GPa.

5. The bulk block of claim 1, wherein the bulk block has a diametral tensile strength of 70 to 80 MPa.

6. The bulk block of claim 1, wherein the bulk block has an average light transmittance of 30 to 40% within a wavelength of 300 to 800 nm, and a maximum water absorption rate of 32 $\mu$g/mm$^3$.

7. The bulk block of claim 1, wherein the polymer is combined with the glass ceramic matrix through silane bonding.

8. The bulk block of claim 1, wherein the polymer is a cured product of a curable organic material selected from (meth)acrylate monomers and oligomers containing unsaturated double bonds.

9. The bulk block of claim 8, wherein the curable organic material is at least one selected from the group consisting of hydroxy ethyl methacrylate (HEMA), 2,2-bis [4-(2-hydroxy-3-methacryloyloxy propoxy) phenyl] propane (Bis-GMA), triethylene glycoldimethacrylate (TEGDMA), diurethanedimethacrylate (UDMA), urethane dimethacrylate (UDM), biphenyldimethacrylate (BPDM), n-tolyglycine-glycidylmethacrylate (NTGE), polyethylene glycol dimethacrylate (PEG-DMA), and oligocarbonate dimethacrylic esters.

10. The bulk block of claim 1, wherein the glass matrix comprises 69.0 to 75.0% by weight of $SiO_2$, 12.0 to 14.0% by weight of $Li_2O$, 2.5 to 10.5% by weight of $Al_2O_3$, 0.12 to 0.22% by weight of ZnO, 1.1 to 2.7% by weight of $K_2O$, 0.1 to 0.3% by weight of $Na_2O$, and 2.0 to 6.0% by weight of $P_2O_5$.

11. The bulk block of claim 10, wherein the glass matrix comprises 2.5 to 3.5% by weight of $Al_2O_3$.

12. A method of manufacturing a bulk block for manufacturing a dental prosthesis, the method comprising:

melting a glass composition comprising 69.0 to 75.0% by weight of $SiO_2$, 12.0 to 14.0% by weight of $Li_2O$, 2.5 to 10.5% by weight of $Al_2O_3$, 0.12 to 0.22% by weight of ZnO, 1.1 to 2.7% by weight of $K_2O$, 0.1 to 0.3% by weight of $Na_2O$, and 2.0 to 6.0% by weight of $P_2O_5$, water-quenching a glass melt to obtain a glass formed body of coarse size, and firstly pulverizing the glass formed body to prepare glass powder with a maximum mean grain size of less than 300 $\mu$m; subjecting the glass powder to crystallization heat treatment in a furnace from room temperature to a maximum temperature of 755 to 810°C for 30 minutes to 6 hours; pulverizing the powder subjected to the crystallization heat treatment to produce glass ceramic powder with a maximum mean grain size of less than 100 $\mu$m; and forming the glass ceramic powder into a predetermined shape.

13. The method of claim 12, wherein the glass composition comprises 2.5 to 3.5% by weight of $Al_2O_3$.

14. A prosthesis, comprising:

a glass ceramic matrix; and a polymer, wherein the glass ceramic matrix is composed of an amorphous glass matrix and a crystalline phase dispersed in the glass matrix, the crystalline phase comprises as a main crystalline phase at least one selected from a leucite crystalline phase and a lithium disilicate crystalline phase, the crystalline phase has a mean grain size of 0.01 to 1.0 $\mu$m, and the polymer is included in an amount of 20 to 40% by weight with respect to the total weight of the prosthesis.

15. The prosthesis of claim 14, wherein the crystalline phase comprises as the main crystalline phase lithium disilicate, and further comprises as a sub-crystalline phase lithium metasilicate.

16. The prosthesis of claim 15, wherein the crystalline phase further comprises as the sub-crystalline phase at least one selected from cristobalite, tridymite, quartz, eucryptite, spodumene, virgilite, petalite, and a mixture thereof.

Fig 1

Amorphous Peak. Total Area = 63870 (1792). Crystallinity = 35.27%(1.65%), FWHM-Threshold = 5.0

Fig 2

Fig 3

Fig 4

Fig 5

Fig 6

Fig 7

Shear bond strength (MPa)

present disclosure

Polymer infiltrated ceramic network

Composite resin nanoceramic

▨ Acid etching
▧ Sand blasting

Fig 8

Fig 9

Fig 10

Fig 11

Fig 12

| Coffee 1 week $\triangle E*$ | | 1 | 2 | 3 | 4 | 5 | 6 | Ave. |
|---|---|---|---|---|---|---|---|---|
| | before immersion | 0.94 | 0.77 | 0.6 | 0.83 | 0.7 | 0.75 | |
| | after immersion | 0.8 | 0.81 | 0.62 | 0.76 | 1.22 | 0.75 | |
| | $\triangle E*$ | 0.14 | 0.04 | 0.02 | 0.07 | 0.52 | 0 | 0.13 |

| Coffee 1 month $\triangle E*$ | | 1 | 2 | 3 | 4 | 5 | 6 | Ave. |
|---|---|---|---|---|---|---|---|---|
| | before immersion | 0.99 | 0.75 | 0.79 | 0.99 | 0.72 | 0.75 | |
| | after immersion | 0.84 | 0.88 | 0.65 | 0.98 | 1.24 | 0.86 | |
| | $\triangle E*$ | 0.15 | 0.13 | 0.14 | 0.01 | 0.52 | 0.11 | 0.18 |

## INTERNATIONAL SEARCH REPORT

| International application No. |
|---|
| **PCT/KR2022/009033** |

### A.    CLASSIFICATION OF SUBJECT MATTER

**A61C 13/00**(2006.01)i; **A61C 13/083**(2006.01)i; **C03C 4/00**(2006.01)i; **C03C 10/00**(2006.01)i; **C08L 33/14**(2006.01)i; **C08L 35/02**(2006.01)i; **C08K 3/40**(2006.01)i; **C03B 32/02**(2006.01)i; **A61K 6/833**(2020.01)i

According to International Patent Classification (IPC) or to both national classification and IPC

### B.    FIELDS SEARCHED

Minimum documentation searched (classification system followed by classification symbols)

A61C 13/00(2006.01); A61C 13/083(2006.01); A61K 6/00(2006.01); A61K 6/02(2006.01); A61K 6/027(2006.01); A61K 6/04(2006.01); A61K 6/831(2020.01); C03C 10/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 치과(dental), 보철물(prosthetic), 벌크(bulk), 블록(block), 글라스-세라믹(glass-ceramic), 고분자(polymer), 리튬 디실리케이트(lithium disilicate)

### C.    DOCUMENTS CONSIDERED TO BE RELEVANT

| Category* | Citation of document, with indication, where appropriate, of the relevant passages | Relevant to claim No. |
|---|---|---|
| X | KR 10-2020-0098346 A (HASS CORPORATION) 20 August 2020 (2020-08-20)<br>See paragraphs [0022]-[0029], [0038], [0042] and [0045]; and claims 1, 3, 4 and 7-8. | 1,4-9,14 |
| Y | | 2-3,10-13,15-16 |
| X | KR 10-2015-0043633 A (HASS CORPORATION) 23 April 2015 (2015-04-23)<br>See paragraphs [0020], [0045] and [0051]-[0053]; and claims 1-2. | 12-13 |
| Y | | 2-3,10-13,15-16 |
| DA | KR 10-1682542 B1 (HASS CORPORATION) 06 December 2016 (2016-12-06)<br>See entire document. | 1-16 |
| A | KR 10-2012-0131603 A (VERICOM CO., LTD. et al.) 05 December 2012 (2012-12-05)<br>See entire document. | 1-16 |

☑ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **21 September 2022** | **21 September 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2022/009033**

### C.    DOCUMENTS CONSIDERED TO BE RELEVANT

| Category* | Citation of document, with indication, where appropriate, of the relevant passages | Relevant to claim No. |
|---|---|---|
| PY | WO 2022-065560 A1 (HASS CO., LTD.) 31 March 2022 (2022-03-31)<br>See paragraphs [0004]-[0007] and [0022]-[0025]; and claims 1-17. | 2-3,10-13,15-16 |

Form PCT/ISA/210 (second sheet) (July 2019)

**INTERNATIONAL SEARCH REPORT**
Information on patent family members

International application No.

**PCT/KR2022/009033**

| Patent document cited in search report | | | Publication date (day/month/year) | Patent family member(s) | | | Publication date (day/month/year) |
|---|---|---|---|---|---|---|---|
| KR | 10-2020-0098346 | A | 20 August 2020 | CN | 111770747 | A | 13 October 2020 |
| | | | | EP | 3714862 | A1 | 30 September 2020 |
| | | | | JP | 2020-132632 | A | 31 August 2020 |
| | | | | JP | 7009526 | B2 | 25 January 2022 |
| | | | | KR | 10-2228118 | B1 | 16 March 2021 |
| | | | | US | 2021-0214483 | A1 | 15 July 2021 |
| | | | | WO | 2020-166869 | A1 | 20 August 2020 |
| KR | 10-2015-0043633 | A | 23 April 2015 | EP | 3059214 | A1 | 24 August 2016 |
| | | | | EP | 3059214 | B1 | 01 August 2018 |
| | | | | KR | 10-1524482 | B1 | 02 June 2015 |
| | | | | US | 2015-0104655 | A1 | 16 April 2015 |
| | | | | US | 9731995 | B2 | 15 August 2017 |
| | | | | WO | 2015-056900 | A1 | 23 April 2015 |
| KR | 10-1682542 | B1 | 06 December 2016 | KR | 10-2016-0112879 | A | 28 September 2016 |
| KR | 10-2012-0131603 | A | 05 December 2012 | KR | 10-1325281 | B1 | 08 November 2013 |
| WO | 2022-065560 | A1 | 31 March 2022 | CN | 114276021 | A | 05 April 2022 |
| | | | | EP | 3974396 | A1 | 30 March 2022 |
| | | | | JP | 2022-055274 | A | 07 April 2022 |
| | | | | KR | 10-2022-0043948 | A | 06 April 2022 |
| | | | | US | 2022-0096212 | A1 | 31 March 2022 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101682542
- KR 101609291
- KR 102122202
- KR 102228118
- US 7807227 B
- JP 4636514 B